# EUROPEAN PATENT SPECIFICATION

(11) **EP 2 825 239 B1**
(45) Date of publication and mention of the grant of the patent: **03.08.2022**
(21) Application number: 13760437.7
(22) Date of filing: 13.03.2013
(51) Int. Cl.: A61L 29/06, A61L 29/16, A61L 29/10, A61L 29/14, A61M 25/00

(54) **CATHETER HAVING ANTIMICROBIAL COATING**
KATHETER MIT ANTIMIKROBIELLER BESCHICHTUNG
CATHÉTER AYANT UN REVÊTEMENT ANTIMICROBIEN

(30) Priority: 13.03.2012 US 201261610344 P
(43) Date of publication of application: 21.01.2015
(73) Proprietor: PFM Medical, Inc., Carlsbad, CA 92008 (US)
(72) Inventor: KERR, Marshall, Carlsbad, CA 92008 (US)
(74) Representative: London IP Ltd
(86) International application number: PCT/US2013/031095
(87) International publication number: WO 2013/138534

(56) References cited:
- EP-A2- 0 799 623
- WO-A1-2011/014201
- WO-A1-2013/033023
- DE-A1- 3 228 849
- RU-A- 2009 125 467
- US-A1- 2010 204 777
- US-B1- 6 726 651
- US-B2- 6 629 969

## Description

### BACKGROUND OF THE INVENTION

### 1. Field of the Invention

The present invention refers to catheters. More specifically, the invention relates to a catheter, such as a periphery inserted central catheter (PICC), having an anti-microbial coating on one or both ends to reduce the chance of pathogens being introduced to the insertion site or entering a patient=s body during insertion, removal, and operative use of the PICC.

Infection of the tissue at and adjacent to an insertion site, and/or the vascular system of a patient may easily occur during the insertion, use, and removal of a PICC or other type catheter from the body of a patient. The patient=s skin, initially punctured by a needle or other intravascular access instrument to allow the insertion of a catheter through the skin and into a vein or artery, may be exposed to infectious agents such as bacteria, viruses, fungi, and other infectious agents disposed on and about the exterior surface of the skin of a patient, on conventional catheter, may easily be drawn into the insertion site through a contact with the exterior surface of a catheter with the skin, during the insertion, implementation, and removable of the catheter past the skin barrier. This invasion of infectious agents, can lead to inflamation and cell destruction in the tissues surrounding the puncture, or other remote infection sites, should an infectious agent carried on the catheter reach the patient=s blood stream. The present invention provides a solution to the shortcomings of such conventional catheters through the provision of an infectious agent preventing antimicrobial coating, disposed the skin-contacting surfaces of and catheter used in combination therewith, to thwart the communication of infectious agents such as bacteria and viruses into the flesh below an insertion site.

### 2. Prior Art

Intravascular catheters, such as periphery inserted central catheters (PICC), are forms of intravenous access often employed for chemotherapy regimes, antibiotic therapy, prenatal nutrition, and other treatments requiring vascular communication from outside the body of the patient. These types of catheters or conduits are often used for extended periods of time which can extend to 30 days or more. In use, the PICC, or other type catheter, must first be communicated into a vascular conduit or peripheral vein such as the cephalic vein, basilic vein, or brachial vein. Once the catheter providing the fluid conduit is inserted through the skin and into the patient=s vascular system, it is advanced toward the heart to a positioning point. At this positioning point, generally, the needle tip is in a positioned communication with the cavoatrial junction.

Conventionally, insertion of the catheter through the skin and surrounding tissue of the vascular system is accomplished by a multi step process. This process is performed by a medical professional employing a plurality of medical components and instruments or tools. A commonly employed tool for piercing the skin and tissue of a patient is known as an introducer. The introducer is inserted through the skin and tissue and a distal end placed in communication with the target artery or vein of the vascular system of the patient. Once the introducer is properly engaged, it provides a conduit for the physician to easily insert the catheter axially therethrough, once the target vein has been located.

To properly insert and position the distal end of the catheter, for communicating and delivering the medication directly to the vascular system of the patient, first, a tourniquet or similar device is applied to the arm or leg of the patient at a position above the anticipated insertion site. This constricts blood flow thereby providing means to distend the veins so as to allow the medical professional to target a vein or artery of choice.

Subsequent to targeting the vein or artery, an introducer needle, often called a Aseldinger needle, engaged to a forward portion of a syringe, is inserted into the target vein and the tourniquet is released. The syringe is then removed and the user seals or places their thumb over the end of the needle to prevent blood loss or an intake of an air embolism.

Employing a flexible guidewire, the distal end of the wire is then inserted into the needle until only the proximal end of the guidewire is visible. The guidewire is advanced with a forward motion into and past the needle hub into the target vein. With the guidewire in place the needle is removed.

The introducer, including sheath and dilator, is thread over the proximal end of the guidewire and into the target vein. Often a physician will nick the skin with a safety scalpel for easier advancement of the dilator. Once a proper catheter length has been determined, the dilator is removed from the sheath. Further, the distal tip of the catheter is inserted into and through the sheath until the catheter tip is correctly positioned in the target vein. The tear-away sheath is then removed by pulling it out of the vessel while simultaneously splitting the sheath. From here, adjustments can be made to the catheter as needed.

In a subsequent step, the distal tip of the catheter is inserted into and through the sheath until the catheter tip is correctly positioned within the target vein. The tear-away sheath is then removed by pulling it out of the vessel while simultaneously splitting the sheath. From here, adjustments can be made to the positioning of the distal end of the catheter. Once properly positioned, it is quite common for the proximal end of the catheter to remain protruding from the insertion site. The proximal end may then be connected to valves for fluid drainage or introduction of medicine or other fluid into the catheter as needed. As one can clearly see, there are numerous deliberate and precise actions which take place at an insertion site in such a procedure so to successfully position the catheter. Such being, namely, the positioning of the distal end of the catheter past the insertion site to the correct depth into the target vein to allow a sufficient length of the proximal end of the catheter to remain extending from the insertion site past skin barrier of the patient. Although modern medicine provides means to sterilize the catheter and other components and they are provided in sterile packages, there is a great chance of driving surface area bacteria into the insertion site upon the exterior surface of the catheter as it passes through the skin and into the intended blood vessel. The exterior surface of the distal end of the catheter introduced past the skin barrier into the insertion site, provides a surface carrier for such infectious agents as it passes through the skin barrier and underlying tissue and communicates through the vessel wall and into the intended blood vessel. Further, the exterior surface of the proximal end of the catheter remaining protruding from the insertion site provides yet another surface carrier for such infectious agents.

As such, the distal end during insertion, and the proximal end being adjacent to and communicating through the insertion site, may allow bacteria to be introduced into the patient.

As such, there is a continuing unmet need for a catheter configured for use in a method which provides enhanced protection against the communication of pathogens and infectious agents into the body of the patient. Such a device enabling such enhanced preventions should provide additional means beyond conventional sterilization techniques, to prevent such infectious agents from entering the underlying tissue and vascular system of a patient through an insertion site when medical devices are communicated through the skin of a patient. Such a device should provide anti pathogenic or antimicrobial surface property to exterior surfaces of the catheter employed in combination therewith, to thereby extinguish or inhibit the growth of pathogens communicating the infectious agents to underlying tissue upon a contact with the respective exterior surfaces.

WO2013/033023A1 discloses the exterior surfaces of a catheter being coated with titanium to increase bio-compatibility.

DE3228849A1 describes a medical device having an adhesion-promoting layer consisting of elemental carbon or polyurethane. Over the layer is a metal ion-donating layer of carbon or titanium. Further layers of ion-donating material and a fabric-compatible substance made of elemental carbon or a polymer are applied.

With respect to the above description, before explaining at least one preferred embodiment of the herein disclosed invention in detail, it is to be understood that the invention is not limited in its application to the details of construction and to the arrangement of the components in the following description or illustrated in the drawings.

### SUMMARY OF THE INVENTION

According to this invention there is provided a catheter (12) comprising:
at least one interior lumen defined by a side wall having an exterior circumferential surface between a first end (22) of said catheter and a second end (20) of said catheter;
a titanium surface area (17) on said circumferential surface, characterised by said titanium surface area extending a distance toward a central portion (I) of said catheter from one or both of said first end and said second end (20) and defining means for inhibiting pathogens, and means for enhancing lubricity with tissue contacting said circumferential surface, thereby providing prevention of tissue adherence thereto, and
an anti-pathogenic and/or anti-microbial surface (16) coating over said titanium surface area circumferential surface at one or both of said first and said second end.

Preferred features are defined in the claims dependent on claim 1.

The device herein disclosed and described provides a solution to the shortcomings in prior art and achieves the above noted goals through the provision of an antimicrobial exterior surface of catheter devices employed to communicate through the skin and to the intended blood vessel or other target where skin piercing is required. An antimicrobial surface area for a distance sufficient to communicate through the skin and above the exterior surface of the patient=s skin is formed upon one or both distal ends of a catheter, such as a PICC. In this fashion, during any communication of the catheter device through the skin, and into the patient, bacteria, viruses and other infectious occupants of the exterior and any underlying layers of skin, will come into direct contact with the antimicrobial coating.

Further, in other preferred modes, the catheter device may employ bio-compatible surface coating in combination with the antimicrobial exterior surface coating. By positioning a thin surface area of titanium on the exterior circumferential surface of polyurethane and other catheters in combination with an antimicrobial coating, the device and method herein provide a means to render conventional catheters into catheters with increased bio-compatibility as well as prevent the introduction of surface bacteria into the insertion site. Additionally, one skilled in the art will additionally find that such increased bio-compatibility may additionally be accomplished by providing a surface area of titanium on the interior surface and ends of the catheter in combination with the above noted exterior surface layer.

Titanium, modernly, has been found in most humans to generally elicit little to no immune system response. Additionally, as a general rule, it lacks any toxic or other injurious effect on the patient=s body. Consequently, it is frequently employed for implants as it can be left in the body for extended periods of time without discomfort, infection or irritation. As such, titanium has replaced stainless steel for long term oral implants, and many joint and bone implants where strength is a requirement and long term body acceptance is a must. Such titanium implants are generally formed in a manner to take advantage of the material=s light weight but great strength and resistence to bending and deterioration.

Antimicrobial material can be any material suitable for the intended purpose and having antimicrobial properties may be employed. As defined in the claims, is disclosed a combination of materials in a coating extending the distal end of the catheter which is first introduced through the skin to a portion of the length of the catheter, as well as the proximal end of the catheter which remains protruding from the insertion site, to a length positioning the coated surface above and below the skin layer once fully inserted. Such materials include one or a combination of antimicrobial materials from a group including nitrofurazone-coated silicone or silver or silver ions or silver nano-particles in a coating, or copper or copper bearing materials in a coating, chlorhexidine incorporated hydroxylapatite coatings, chlorhexidine-containing polylactide coatings on an anodized surface, and polymer and calcium phosphate coatings with chlorhexidine, and aluminum and aluminum ions. However, in other preferred modes the catheter may be impregnated or otherwise formed with antimicrobial materials and properties.

As used in the claims to describe the various inventive aspects and embodiments, "comprising" means Aincluding, but not limited to, whatever follows the word "comprising". Thus, use of the term "comprising" indicates that the listed elements are required or mandatory, but that other elements are optional and may or may not be present. By "consisting of is meant including, and limited to, whatever follows the phrase "consisting of". Thus, the phrase "consisting of" indicates that the listed elements are required or mandatory, and that no other elements may be present. By "consisting essentially of" is meant including any elements listed after the phrase, and limited to other elements that do not interfere with or contribute to the activity or action specified in the disclosure for the listed elements. Thus, the phrase "consisting essentially of" indicates that the listed elements are required or mandatory, but that other elements are optional and may or may not be present depending upon whether or not they affect the activity or action of the listed elements.

It is an object of the invention to provide a catheter device for communicating a distal end through the skin layer of a patient to an interior target, where the proximal end remains protruding from the incision site, which has an antimicrobial coating portion adjacent to one or both of the distal and proximal ends, such as PICC.

### BRIEF DESCRIPTION OF DRAWING FIGURES

Figure 1 shows a view of a catheter device having antimicrobial coatings on portions of one or both of the proximal and distal ends.
Figure 2 shows a perspective view of an end of the catheter device depicting a multi lumen catheter tube having antimicrobial coating for a distance from the end toward the center. (not part of the invention)
Figure 3 is a cross section view of one mode of the device of figure 2, showing a multi lumen catheter having an antimicrobial exterior coating, along cut line AA from figure 2. (not part of the invention)
Figure 4 is a cross section view of another preferred mode of the device of figure 1, showing a multi lumen catheter having antimicrobial and titanium exterior coatings.
Figure 5 shows a mode of the catheter engaged in sterile packaging.
Figure 6 shows a view of another mode of the device employing a spray bottle or other suitable means for application allowing the user to adapt existing catheters which may or may not have titanium surface layers, with an applied antimicrobial surface area in an evaporative adhesive carrier.

### DETAILED DESCRIPTION OF THE PREFERRED

### EMBODIMENTS OF THE INVENTION

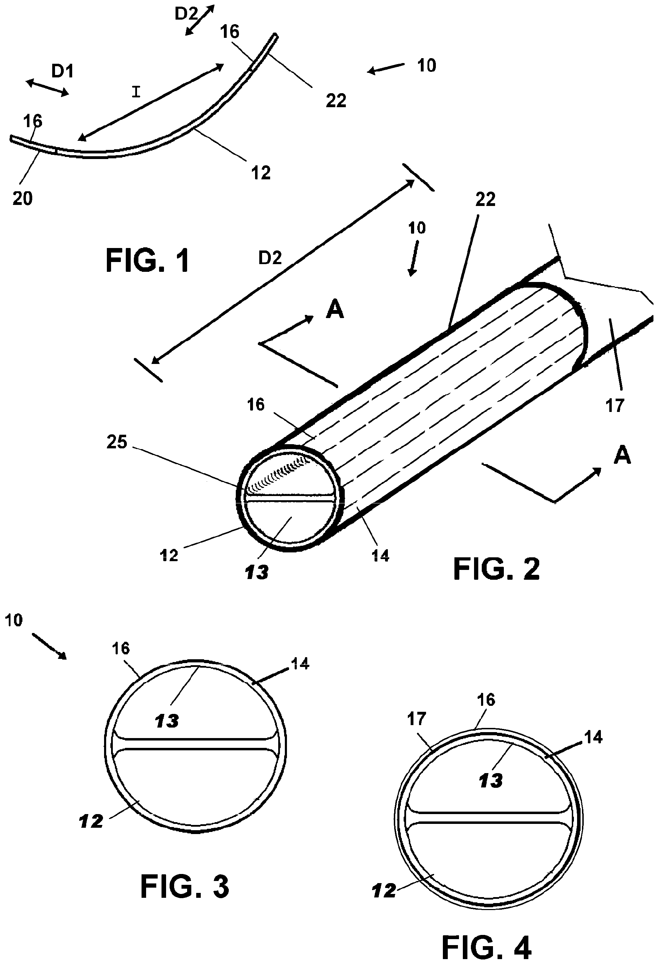

Now referring to drawings in figures 1-6, wherein similar components are identified by like reference numerals, there is seen in FIG 1 a view of the device 10 providing a catheter 12 having titanium 17 combined with antimicrobial coated surfaces 16 at one or both of the distal 20 and proximal 22 ends. In some modes the entire exterior surface may be form a titanium 17 surface area, and in a minimum mode, at least one and preferably both ends have a titanium 17 surface area in a range from 2.54cm to 25.4cm (1 to 10 inches) from the end toward a central portion of the catheter.

At the distal end 20, the coating 16 may extend from the terminating endwall 25 a distance D1 on the exterior surface 14. Further, at the proximal end 22, the coating 16 may extend from the opposite terminating endwall 25 a distance D2 on the exterior surface 14. As noted previously, the distal end 20 is inserted through the skin barrier and is a potential carrier for infectious agents, while the proximal end 22 will remain protruding from the insertion site and skin barrier, as needed for operative connection of other instruments for the introduction of draining of fluid. Thus it is to be understood that the distances D1, D2 employing such as coating 16, may differ in length as needed for the intended purposes of the device. Those skilled in the art may immediately discover suitable lengths for these distance, and are anticipated.

Further, the catheter 12 has an intermediate portion AI extending from the coating 16 area of the proximal end 22 to the coating 16 area of the distal end 20. This intermediate portion I, may or may not include the coating 16 material in its application as disclosed with the operative use of this invention.

The device disclosed herein can be formed of conventional materials such as polyurethane, silicone, or polytetrafluoroethylene ("PTFE"). However, it can also be formed of any material suitable for use in combination with the disclosed coating of an antimicrobial material for the purposes set forth in this disclosure. Again, it must be noted that the device 10 can alternatively be formed all, or partially, itself of an antimicrobial impregnated material in a solid solution, or any other means to impart antimicrobial properties therein to communicate with the cicumfrential surface.

It is to be noted that the antimicrobial coating may be applied by any conventional means known in the art such as vacuum chamber coating, plasma coating, employment of an antimicrobial material in an impregnated polymer or other carrier used as coatings, or impregnating the proximal and distal ends of the catheter itself. While titanium is a particularly favored antimicrobial material herein, because experimentation has shown it to encourages the attraction of a fluid coating to the circumferential surface, and thus it has been found to be a means for encouraging lubricity to the outside of the catheter to prevent tissue adherence, the antimicrobial materials also includes one or a combination of antimicrobial materials prreferably from a group including nitrofurazone-coated silicone or silver or silver ions or silver nano-particles, copper or copper bearing materials in a coating. Other antimicrobial materials may be also placed adjacent in combination with the titanium such as one or a combination of, chlorhexidine incorporated hydroxylapatite coatings, chlorhexidine-containing polylactide coatings on an anodized surface, and polymer and calcium phosphate coatings with chlorhexidine, in a coating or mixed in a polymer coating.

It can be an advantage of the present invention to provide a antimicrobial coated surface area 16 of the distal end 20 of the catheter 12 using titanium in combination with the other noted antimicrobials herein, in order to reduce or eliminate contamination by pathogens at the insertion site during operative insertion of the catheter 12. Such reduction would continue during the period the catheter remains implanted in the patient and, the titanium surface area will provide enhanced lubricity to prevent tissue adherence thereto.

In addition and preferred, the first or proximal end 22 is anti-microbial coated since it protrudes from the insertion site communicating through the patient's skin, once the insertion procedure is complete. The placement of the coating on the proximal end which communicates through the skin surface will inhibit pathogen travel along the catheter surface and below the skin surface of the patient which is the first barrier to such intrusions.

Further, (not part of the invention), the entire length of the exterior surface 14 of the catheter 12 may additionally employ antimicrobial materials thereon singularly or in combination, or may have a titanium surface alone since it provides a means for encouraging lubricity. Alternatively, the titanium surface area on the entire cicumfrential surface may be employed in combination with one or a combination of the other noted antimicrobials herein.

Figure 2, discloses (but not part of the invention) the coating as it would be for a distance on one or both the proximal and distal ends of the catheter. Shown in the elevated and cross sectional views of FIG 2 and FIG 3 respectively, the device 10 comprising a catheter 12 having a surface area of antimicrobial material 16 thereon which is coated, adhered, impregnated, or otherwise formed thereon in a manner to communicate directly with the exterior surface area 14 of the catheter 12 on one or both ends. The catheter 12 may be polyurethane or silicone, polyethylene or any other material known in the art for catheters and similar type tubing suited for receiving an antimicrobial coating at one or both ends.

As defined in the claims, a titanium surface area or portion, may preferably be included in combination with one or more of the other noted antimicrobial materials on one or both ends and is a particular favored mode with both pathogenic killing action and lubricity enhancement and within the scope of the device 10. The titanium surface area or surface portion is combined with one or a combination of the other antimicrobial materials from the group thereof including chlorhexidine incorporated hydroxylapatite coatings, chlorhexidine-containing polylactide coatings on an anodized surface, and polymer and calcium phosphate coatings with chlorhexidine. This allows the titanium exterior surface area to provide a means for inhibiting a rejection reaction by the body's protective actions such as attacks by white blood cells, to render the long term or even short term placement of the catheter for such procedures as a PICC line, easier on the patient's body. Further, titanium so situated, in addition to enhanced lubricity, has been found to have antimicrobial and anti pathogenic properties.

While shown as a double lumen catheter, those skilled in the art will realize that catheters come with varying numbers of internal lumens from one lumen to multiples and the depicted dual lumen catheter 12 is for illustration purposes only since the invention herein is applicable to all catheters and tubes which may be employed in medical uses for insertion into a patient.

FIG 4 depicts a cross section of the mode according to the invention: the device 10 employing both a titanium and antimicrobial coating. The titanium surface area can be along the entire exterior surface, and the other materials antimicrobial and anti pathogenic coating may be at one or both ends of the catheter and be one or a combination from the noted group above.

It is preferred that the catheter 12 maintains its flexibility as needed for intravenous insertion and such is provided insofar as the titanium surface area 17 is substantially thin and since titanium by nature is more ductile than other metals. In a particularly preferred mode, the titanium surface area 17 on the exterior surface 14 of the catheter 12 is in the order of magnitude of 2 microns or less. In this manner, the catheter 12 employs the benefits of antimicrobial properties and anti pathogenic properties as well as the bio-compatibility and other benefits associated with the antimicrobial surface area 16, and titanium surface area 17, without compromising the flexibility of the structure needed for advancing the device 10 through the often serpentine like blood vessels to place the distal end in the desired location. Further, when wet, the titanium surface provides a means to enhance lubricity and overcome friction of tissue against the exterior of the catheter 12, and to prevent attachment of tissue to the outside of the catheter 12 over the longer time periods of insertion such as with a PICC line.

The titanium surface area 17 is preferably formed by conventional methods such as coating, spraying, thermoforming, film engaging or extruding the catheter using a mix of polymeric material and titanium oxide in a mix, or shrink wrapping or vacuum forming a titanium sleeve thereon, or other means known in the art. Further, it must be noted that in order to adhere or otherwise form the titanium surface area 17 on some types of catheters 12 those skilled in the art may employ various other alloys of titanium better suited for varying types of tubing material.

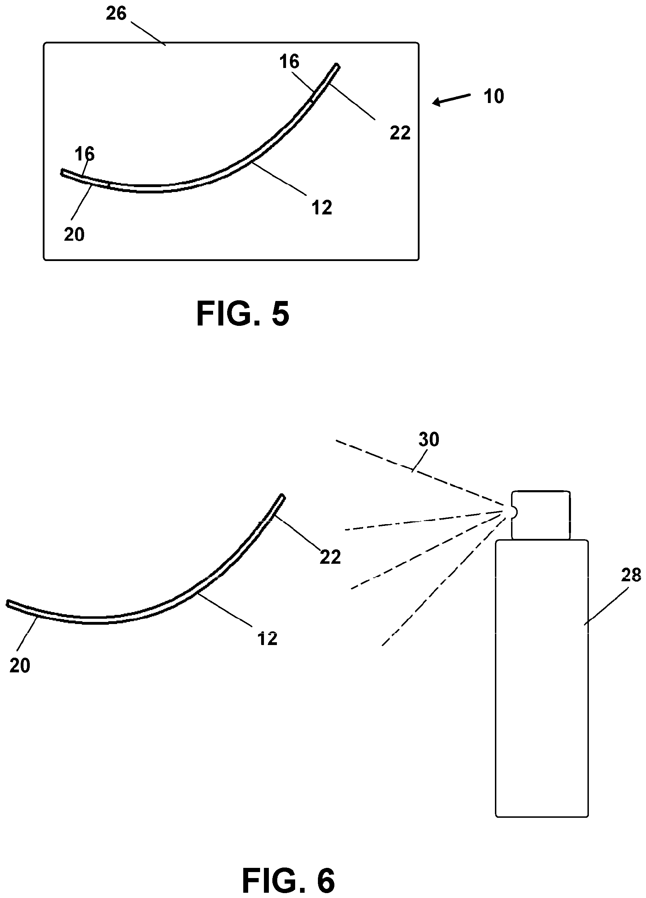

FIG 5 shows a mode of the device 10 with the catheter 12 engaged within sterile packaging 26 as is conventional in the art. The packaging 26 may have a tear-away corner allowing the user to open the packaging 26 and retrieve the catheter 12.

FIG 6 shows yet another mode of the disclosed device 10 providing the user with a means for adapting existing unprotected catheters 12 with an antimicrobial coating. Currently, this is provided by a spray bottle 28 or other container for discharging a mixture of a curing carrier such as polyethylene or another polymeric material which will cure when exposed to air or with addition of a curing agent. The antimicrobial material 30 mixed to dispense with the polymeric or other curing carrier, in a spray, stream, atomized spray, or the like, for the purpose of user applying an area covered with a cured solid solution of polymeric material and antimicrobial agent such as silver nitrate which will communicate silver ions to the antimicrobial surface coating 16 formed by the dispensed mixture 30 on the desired unprotected surfaces of catheter 12. However, it is noted that other modes are envisioned wherein the antimicrobial material is provided as a liquid, gas, salve, balm, or the like which allows the user to apply a coating onto the desired instrument.

It must be noted that it is within the scope of the invention that the terminating endwall surfaces 25 of the proximal 20 and distal end 22 may additionally employ antimicrobial 16 and titanium 17 coatings as well.

All such modifications and variations and substitutions are included within the scope of the invention as defined by the following claims.

## Claims

1. A catheter (12) comprising:
at least one interior lumen defined by a side wall having an exterior circumferential surface between a first end (22) of said catheter and a second end (20) of said catheter;
a titanium surface area (17) on said circumferential surface, **characterised by** said titanium surface area extending a distance toward a central portion (I) of said catheter from one or both of said first end and said second end (20) and defining means for inhibiting pathogens, and means for enhancing lubricity with tissue contacting said circumferential surface, thereby providing prevention of tissue adherence thereto, and
an anti-pathogenic and/or anti-microbial surface (16) coating over said titanium surface area circumferential surface at one or both of said first and said second end.

2. A catheter as claimed in claim 1, wherein the titanium surface area extends along the entire exterior and/or interior surface of said lumen.

3. A catheter as claimed in any preceding claim, wherein the anti-pathogenic and/or anti-microbial surface coating is one or a combination of anti-microbial materials from a group including nitrofurazone-coated silicone or silver or silver ions or silver nano-particles in a coating, or copper or copper bearing materials in a coating, chlorhexidine incorporated hydroxylapatite coatings, chlorhexidine-containing polylactide coatings on an anodized surface, and polymer and calcium phosphate coatings with chlorhexidine, and aluminum and aluminum ions.

## Patentansprüche

1. Ein Katheter (12) umfassend:
Mindestens ein Innenlumen, definiert durch eine Seitenwand mit einer äußeren Umfangsfläche zwischen einem ersten Ende (22) dieses Katheters und einem zweiten Ende (20) des Katheters;
einen Titan-Oberflächenbereich (17) auf dieser Umfangsfläche, **gekennzeichnet durch** diesen Titan-Oberflächenbereich, der sich eine Distanz zu einem zentralen Abschnitt (I) dieses Katheters von einem oder beiden des ersten Endes oder des zweiten Endes (20) erstreckt und eine Vorrichtung definiert, die Krankheitserreger hemmt, und eine Vorrichtung, die die Schmierfähigkeit des Gewebes im Kontakt mit dieser Umfangsfläche vergrößert und dadurch verhindert, dass das Gewebe daran festklebt, und
eine antipathogene und/oder antimikrobielle Oberfläche (16), die die Umfangsfläche dieses Titan-Oberflächenbereichs an einem oder beiden des ersten und zweiten Endes beschichtet.

2. Ein Katheter nach Anspruch 1, wobei sich der Titan-Oberflächenbereich entlang der gesamten Außen- und/oder Innenfläche dieses Lumen erstreckt.

3. Ein Katheter nach einem der vorhergehenden Ansprüche, wobei die antipathogene und/oder antimikrobielle Oberflächenbeschichtung ein oder eine Kombination aus antimikrobiellen Materialien aus der Gruppe bestehend aus Nitrofural-beschichtetem Silikon oder Silber oder Silberionen oder Silber-Nanopartikeln in einer Beschichtung, oder Kupfer oder kupferenthaltende Materialien in einer Beschichtung, Chlorhexidin-inkorporierte Hydroxylapatit-Beschichtungen, Chlorhexidin-enthaltende Polylactid-Beschichtungen auf einer eloxierten Oberfläche, und Polymer und Calciumphosphat-Beschichtungen mit Chlorhexidin, sowie Aluminium und Aluminiumeisen ist.

## Revendications

1. Cathéter (12) comprenant :
au moins une lumière intérieure définie par une paroi latérale présentant une surface circonférentielle extérieure entre une première extrémité (22) dudit cathéter et une seconde extrémité (20) dudit cathéter ;
une surface spécifique en titane (17) sur ladite surface circonférentielle, **caractérisée en ce que** ladite surface spécifique en titane s'étend sur une distance vers une partie centrale (I) dudit cathéter à partir de l'une ou des deux parmi ladite première extrémité et ladite seconde extrémité (20) et définit un moyen destiné à inhiber les agents pathogènes et un moyen destiné à améliorer le pouvoir lubrifiant avec le tissu en contact avec ladite surface circonférentielle, empêchant ainsi l'adhérence du tissu à celle-ci, et
un revêtement de surface (16) antipathogène et/ou antimicrobien sur ladite surface circonférentielle de la surface spécifique en titane à l'une ou aux deux parmi ladite première et ladite seconde extrémité.

2. Cathéter selon la revendication 1, dans lequel la surface spécifique en titane s'étend le long de toute la surface extérieure et/ou intérieure de ladite lumière.

3. Cathéter selon l'une quelconque des revendications précédentes, dans lequel le revêtement de surface antipathogène et/ou antimicrobien est un matériau ou une combinaison de matériaux antimicrobiens provenant d'un groupe comprenant le silicone revêtu de nitrofurazone ou l'argent ou les ions argent ou les nanoparticules d'argent dans un revêtement, ou le cuivre ou les matériaux portant du cuivre dans un revêtement, les revêtements d'hydroxylapatite incorporés à la chlorhexidine, les revêtements de polylactide contenant de la chlorhexidine sur une surface anodisée et les revêtements de polymère et de phosphate de calcium comportant la chlorhexidine, et l'aluminium et les ions aluminium.
